# EUROPEAN PATENT APPLICATION

(11) **EP 4 617 378 A1**
(43) Date of publication of application: **17.09.2025**
(21) Application number: 24163779.2
(22) Date of filing: 15.03.2024
(51) Int. Cl.: C12Q 1/6804, C12Q 1/682, C12Q 1/6841

(54) **AFFINITY REAGENT AND METHOD FOR ANALYSING A BIOLOGICAL SAMPLE**

(71) Applicant: Leica Microsystems CMS GmbH, 35578 Wetzlar (DE)
(72) Inventor: Alsheimer, Soeren, 35578 Wetzlar (DE)
(74) Representative: Schaumburg und Partner Patentanwälte mbB

(57) **Abstract**

In a first aspect an affinity reagent (102, 202) for analysing a biological sample is provided. The affinity reagent (102, 202) comprises a binding backbone (108, 204) and a barcode oligonucleotide (110) attached to the binding backbone (108, 204). The binding backbone (108, 204) is configured to specifically bind to a target analyte (106). The barcode oligonucleotide (110) comprises at least one barcode sequence and a set of primer sequences configured for isothermal amplification. In another aspect a method for analysing a biological sample with the affinity reagent (102, 202) is provided.

## Description

### Technical field

The invention relates to an affinity reagent for analysing a biological sample. In further aspects, a method for analysing the biological sample with a marker comprising the affinity reagent is provided.

### Background

The use of optically detectable markers has transformed the detection of target analytes in biological samples, with fluorescent labelling and imaging playing a vital role in proteomic research. By labelling specific molecules, such as proteins or nucleic acids, with fluorescent dyes, researchers can visualise and quantify their presence within complex biological samples. This approach not only enables the identification of specific biomolecules with high sensitivity and precision but also allows for the tracking of dynamic cellular processes and interactions. Fluorescent markers offer the advantage of multiplexing, where multiple targets can be simultaneously detected using different fluorescent dyes or colours, making them indispensable tools in studying complex biological systems.

However, detecting low abundance proteins in spatial biology applications remains difficult, primarily due to several key challenges related to dynamic range, background noise, and sensitivity of detection methods. The dynamic range of protein expression levels within biological samples is significant, with high-abundance proteins overshadowing the presence of low-abundance ones. This wide range often makes it difficult to visualise and quantify low-abundance proteins accurately, as conventional detection methods may saturate at high concentrations and miss signals in the lower range. This problem is further exacerbated when working with complex tissues or cellular environments, where the presence of numerous other biomolecules can contribute to a high background signal, making it challenging to distinguish the specific low-abundance protein of interest from background noise.

Background noise is another significant hurdle in visually detecting low abundance proteins. Various sources, such as autofluorescence, nonspecific binding of detection reagents, and sample heterogeneity, can introduce unwanted signals that obscure the true signal of the protein of interest. Additionally, the sensitivity of detection methods plays a pivotal role in the success of low-abundance protein detection. Many traditional optical detection techniques may lack the required sensitivity to detect proteins present in low quantities, necessitating the application of more sensitive and specific detection methods, such as mass spectrometry-based approaches.

### Summary

It is an object to provide an affinity reagent that enables optically detecting low abundance target analytes in a biological sample and a corresponding method for analysing a biological sample.

The aforementioned object is achieved by the subject-matter of the independent claims. Advantageous embodiments are defined in the dependent claims and the following description.

In a first aspect an affinity reagent for analysing a biological sample is provided. The affinity reagent comprises a binding backbone and a barcode oligonucleotide attached, in particular covalently attached, to the binding backbone. The binding backbone is configured to specifically bind to a target analyte. The barcode oligonucleotide comprises at least one barcode sequence and a set of primer sequences configured for isothermal amplification.

The affinity reagent enables generating barcoded markers comprising a plurality of bright labels, in particular fluorescent labels, in situ of a biological sample attached to the barcode sequence and amplified barcode sequences. In particular, the barcode sequence may be rapidly amplified or copied based on the isothermal amplification and respective primers to generate amplified barcode sequences. This enables binding of a plurality of labels with a sequence complementary to the (amplified) barcode sequences to the affinity reagent in order to generate a marker. The marker thus comprises a plurality of - in particular fluorescent - labels enabling a bright marker that enables detecting low abundance target analytes. In contrast to a polymerase chain reaction-based amplification heat related damage to the biological sample and the target analyte can be minimised by use of an isothermal amplification method.

The binding backbone of the affinity reagent is preferably a polymeric backbone. In particular, the binding backbone may be nucleic acid based or amino acid based (i.e. peptide backbone). For example, the binding backbone may be an aptamer, an antibody, an antibody fragment, or a nanobody. Further, the binding backbone may alternatively be an artificial polymeric binder such as a synthetic peptide or protein, which is configured to specifically bind a target with high affinity. Such an artificial peptide or protein may be found by phage display screening or designed de novo using computational methods (e.g. matching). An artificial polymeric binder may comprise an artificial backbone like polyethylene glycol, and/or artificial residues, and/or nucleobases. In particular, the term artificial polymeric binder may comprise at least one of the following: a DARPin, an Affimer, a Knottin, an Avimer, a Monobody, an Anticalin, a Fynomer, and an Affibody, which may also be referred to as non-antibody-based binders.

In particular, the binding backbone is configured to specifically bind to the target analyte by its complex structure. The complex structure or conformation of the binding backbone of the affinity reagent may be a secondary, tertiary or quaternary structure of the binding backbone, for example. Thus, the binding backbone of the affinity reagent may preferably have a secondary, tertiary, or quaternary structure. Specifically, individual, discontinuous nucleotides or amino acids of the binding backbone may bind to the target analyte. This is in contrast to continuous nucleotides of a sequence of a nucleic acid binding backbone hybridising to the target analyte, in particular a complementary sequence of the target analyte. This enables binding of the affinity reagent with high specificity, for example to an amino acid based target analyte, such as a protein. In particular, the binding backbone of the affinity reagent, e.g. a (nucleic acid based) aptamer, may bind to its target by paratope-epitope interactions and not via hybridization of complementary nucleic sequences. The binding of the affinity reagent to the target analyte may comprise intermolecular forces such as hydrogen bonding, dipole-dipole interactions, and van der Waals forces.

The barcode oligonucleotide preferably has no complex structure, rather the barcode oligonucleotide has a linear or primary structure that allows the binding of a label that comprises an oligonucleotide with a respective complementary sequence that is complementary to the barcode oligonucleotide of the affinity reagent.

The primer sequences of the set of primer sequences are preferably arranged evenly up- and downstream of the at least one barcode sequence. The isothermal amplification may amplify the at least one barcode sequence in order to generate at least one amplified barcode sequence. This enables targeted amplification of the barcode sequence.

The barcode oligonucleotide may comprise at least one further barcode sequence, which may have a sequence identical or different to the barcode sequence. This enables specifically binding several identical or different, in particular with respect to optical properties, labels to the affinity reagent.

The set of primers sequences preferably comprises at least two primer sequences, more preferably four, six, or eight primer sequences. The isothermal amplification may be a loop-mediated isothermal amplification (LAMP). For loop-mediated isothermal amplification four to six primers recognising six to eight distinct primer sequences may preferably used, for a highly specific amplification reaction
In another aspect a method for analysing a biological sample comprising a target analyte is provided. The method comprising the following steps: introducing an affinity reagent comprising at least one barcode sequence into the biological sample; amplifying within the biological sample the at least one barcode sequence with an isothermal amplification method in order to generate at least one amplified barcode sequence; introducing a plurality of labels into the biological sample, each label comprising an optically detectable moiety and a complementary oligonucleotide comprising a complementary sequence configured to hybridise to the at least one (amplified) barcode sequence; and generating an optical readout of the biological sample.

In particular, the affinity reagent may have the features described above. Preferably, the affinity reagent comprises a binding backbone configured to attach to the target analyte, in particular, as described above.

In the step of introducing the plurality of labels a marker comprising the affinity reagent and the label attached to the barcode oligonucleotide of the affinity reagent is generated. In the step of generating the optical readout, the readout is therefore generated of the biological sample with the marker. Thus, the method enables generating a bright marker in situ of the biological sample and analysing the biological sample by means of the marker.

The optical readout may be generated by means of a microscope, for example.

Preferably, the biological sample may be a cell culture sample, 3D cell culture, organoid, spheroid, tissuoid, tissue section, liquid biopsy, a lysate, or a plasma proteome sample, for example.

The isothermal amplification method is preferably carried out at an essentially constant temperature or in a narrow temperature range, requiring no thermocycling. These temperatures are considerably lower than for polymerase chain reaction amplification methods that are typically carried out in wide temperature ranges with temperatures up to 98 °C during denaturation steps.

Preferably, the isothermal amplification method comprises introducing a set of primers into the biological sample. This enables specifically amplifying the barcode sequence. In particular, the primers of the set of primers are configured to be complementary to a respective sequence of the set of primer sequences of the affinity reagent, in particular of the barcode oligonucleotide. The set of primers preferably comprises four to six primers.

Preferably, the primers of the set of primers are configured to generate hairpin loops. This enables loop-mediated isothermal amplification (LAMP) of the barcode sequence. In particular, the hairpin loops are generated up- and downstream of the at least one barcode sequence. Thus, the hairpin loops may be generated at either 5' or 3' ends of the barcode sequence. In particular, the primers of the set of primers cause the target sequence to loop back on itself in order to generate the hairpin loops. This enables efficiently generating free ends to initiate amplification. Thus, the isothermal amplification method is a loop-mediated isothermal amplification method, in particular.

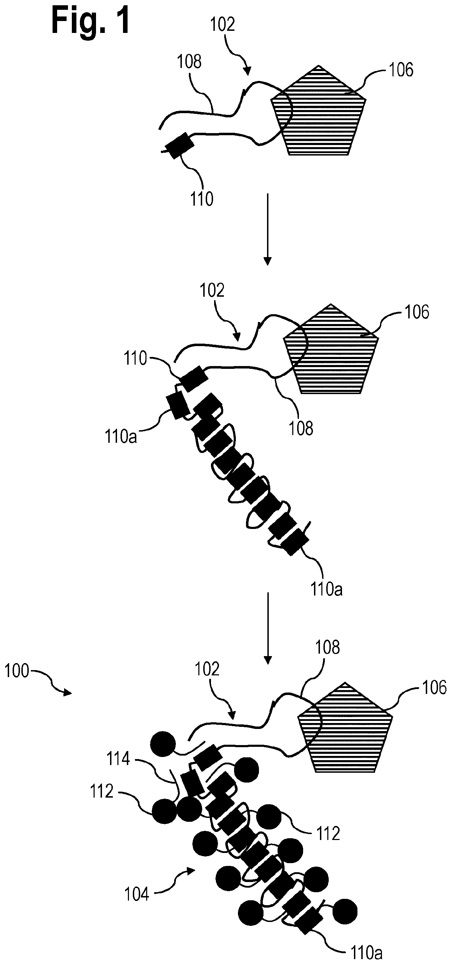

A specific example to generate hairpin loops with a set of primers is given by Notomi et al. 2000 (Notomi T, Okayama H, Masubuchi H, Yonekawa T, Watanabe K, Amino N, Hase T. Loop-mediated isothermal amplification of DNA. Nucleic Acids Res. 2000 Jun 15;28(12):E63 PMID: 10871386) and Podushkina et al., 2019 (Utilizing multiplex fluor LAMPs to illuminate multiple gene expressions in situ), in particular, with reference to Fig. 1 in Podushkina et al., 2019. Generally, loop-mediated isothermal amplification (LAMP) may be utilised for amplification of the barcode sequence.

Preferably, the set of primers comprises at least a first primer with a first region of the first primer being configured to hybridise to at least the barcode oligonucleotide towards a 3' end of the barcode oligonucleotide, and a second region of the first primer being configured to hybridise to the amplified barcode sequence downstream of the first region of the first primer. In particular, the first region of the first primer is at a 3' end of the first primer and the second region is at a 5' end. This enables efficient formation of a hairpin loop at a 3' end of the barcode sequence.

Preferably, the set of primers comprises at least a second primer with a first region of the second primer being configured to hybridise to the amplified barcode sequence towards a 3' end of the amplified barcode sequence, and a second region of the second primer being configured to hybridise to the amplified barcode sequence downstream of the first region of the second primer. In particular, the first region of the second primer is at a 3' end of the second primer and the second region is at a 5' end. This enables efficient formation of a hairpin loop at a 5' end of the barcode sequence.

Preferably, the amplified barcode sequence is a continuous sequence comprising multiple copies of the barcode sequence. This enables binding of a plurality of labels to the affinity reagent.

Preferably, the isothermal amplification method comprises introducing a strand displacing polymerase into the biological sample. This enables efficient generation of the amplified target sequence. Examples of strand displacing polymerases include Phi29 polymerase, Bacillus subtilis DNA polymerase I, T7 DNA polymerase, or T4 DNA polymerase. This enables amplification at low temperature. For example, Phi29 polymerase works at 30°C and is particularly preferred because of low background generation in the biological sample at this temperature; Bacillus subtilis DNA polymerase I works at 37°C - 42°C; T4 and T7 DNA polymerase work at around 37°C.

Preferably, the biological sample is permeabilised and/or expanded, preferably prior to the step of amplifying the barcode sequence. The permeabilisation and/or the expansion of the biological sample may be carried out such that the structure of the biological sample essentially remains unchanged. This allows efficient introduction of amplification reagents into the biological sample.

Preferably, the step of amplifying the at least one barcode sequence is carried out at a substantially constant temperature in a range between 20°C to 75°C, more preferably in a range from 30°C to 65°C, more preferably in a range from 30 °C to 50 °C, even more preferably in a range from 35 °C to 45 °C. This enables carrying out the amplification step without substantial damage to the biological sample due to heat. In particular, the temperature of the amplification step is the temperature the biological sample is at. Preferably, the temperature is held constant at a certain temperature within the range. Preferably, the step of introducing the plurality of labels and of generating the optical readout is similarly carried out at a temperature at or below the temperature of the amplification step.

Preferably, the step of amplifying the at least one barcode sequence is carried out for 0.5 min to 60 min. This enables efficiently generating a sufficient number of copies of the barcode sequence. Preferably the time for carrying out the step is in a range from 1 min to 15 min. This yields an approximate 100- to 10000-fold increase in copy number of the barcode sequence. In particular, this time interval is started after introducing the first set of primers.

Preferably, the step of introducing an affinity reagent comprises introducing a further affinity reagent (preferably having the features of claim 1) comprising at least one further barcode sequence and the step of amplifying within the biological sample comprises amplifying the at least one further barcode sequence with the isothermal amplification method. In particular, the further barcode sequence is different to the at least one barcode sequence. In particular, the further barcode sequence may be amplified by the same or different set of primers than the at least one barcode sequence. This enables efficiently binding different labels to the affinity reagent.

Preferably, the plurality of labels introduced into the biological sample comprises at least a first label with the complementary sequence configured to hybridise to the at least one barcode sequence and at least a second label with a complementary sequence configured to hybridise to the at least one further barcode sequence. Preferably, the first label and the second label differ from each other, for example, in their optical properties such as emission or excitation wavelength, or emission lifetime. This enables generating different, in particular a large number of, optically distinguishable markers for analysing the biological sample.

Preferably, the optically detectable moiety comprises a fluorophore. The label, in particular the fluorophore, may be detected by means of a microscope, for example.

Preferably, in the step of generating the optical readout, at least one of the presence and location of the target analyte is determined in the biological sample. This enables efficient analysis of the biological sample.

Generally, a plurality of markers may be introduced into the biological sample, the markers being specific to respective target analytes, in order to identify a large number of target analytes at the same time. Preferably, the target analyte is identified and/or localised within the biological sample based on the labels of the markers, in particular the labelling moieties, associated with the target analyte in the optical readout.

In a further aspect a marker kit is provided. The marker kit comprises the affinity reagent described above, a set of primers configured to bind to the set of primer sequences, and at least one label comprising a complementary oligonucleotide comprising a complementary sequence configured to hybridise to the barcode sequence. The marker kit enables efficiently generating a bright marker in a biological sample. For example, the marker kit, in particular its components, may be used with the method above.

In particular, the label further comprises an optically detectable moiety, such as a fluorophore. The primers of the set of primers are preferably configured to generate hairpin loops at ends of the barcode oligonucleotide.

Thus, a marker may be generated from the marker kit, wherein the marker comprises the affinity reagent with the label bound to the barcode sequence, as well as amplified barcode sequences. Preferably the marker kit comprises a plurality of labels to bind to the amplified barcode sequences.

Preferably, the kit may further comprise enzymes, such as polymerases, for isothermal amplification of the barcode oligonucleotide, in particular according to the method described above.

### Short Description of the Figures

Hereinafter, specific embodiments are described referring to the drawings, wherein:
- Figure 1: is a schematic view of a marker comprising an affinity reagent and of steps to generate the marker, and
- Figure 2: is a schematic view of a further marker comprising a further affinity reagent.

### Detailed Description

Figure 1 is a schematic view of a marker 100 comprising an affinity reagent 102 and a label 104 and of steps to generate the marker 100. The affinity reagent 102 is configured to specifically bind to a target analyte 106, for example a protein of a biological sample. In particular, the affinity reagent 102 comprises a binding backbone 108 configured to specifically bind to the target analyte 106.

The binding backbone 108 is a nucleic acid based aptamer, for example. Thus, the binding backbone 108 may form or fold into a complex structure or conformation. The complex structure of the backbone 108 of the affinity reagent 102 may be a secondary, tertiary and/or quaternary structure of the backbone 108. Thus, the backbone 108 of the affinity reagent 102 has a secondary, tertiary or quaternary structure, in particular. The affinity reagent 102 binds specifically to the target analyte 106 due to the complex structure of the backbone 108 of the affinity reagent 102. Specifically, individual, discontinuous nucleotides of the nucleic acid of the backbone 108 bind to the target analyte 106. This binding may comprise intermolecular forces such as hydrogen bonding, dipole-dipole interactions, and van der Waals forces. This is in contrast to a nucleic acid hybridising, which is characterised by a continuous sequence of nucleotides of the nucleic acid hybridising to a target analyte, in particular a complementary nucleotide sequence of the target analyte.

The binding of the backbone 108 of the affinity reagent 102 to the target analyte 106 based on the complex structure of the backbone 108 enables particular high affinity binding and specificity to the target analyte 106.

The nucleic acid of the backbone 108 may comprise multiple individual sequences or strands of a nucleic acid. These individual sequences may bind to each other and form a quaternary structure.

The affinity reagent 102 further comprises a barcode oligonucleotide 110. The barcode oligonucleotide 110 may be part of the sequence of the nucleic acid of the binding backbone 108. The label 104 of the marker 100 may comprise a plurality of labelling moieties 112 that are each conjugated to a complementary oligonucleotide 114 that is complementary to a barcode sequence of the barcode oligonucleotide 110. Further, the barcode oligonucleotide 110, in particular the barcode sequence, of the marker 100 is amplified or copied to generate several amplified barcode sequences 110a. Thus, the labelling moieties 112, in particular the complementary oligonucleotide 114, may hybridise specifically to the (amplified) barcode sequences 110, 110a to generate the label 104 of the marker 100, as shown in the bottom view of Fig. 1.

In order to generate the marker 100, the barcode oligonucleotide 110, in particular its barcode sequence, is amplified or copied starting from the affinity reagent 102 comprising only the barcode oligonucleotide 110, as shown in the top view of Fig. 1.

Prior to amplifying the barcode oligonucleotide 110, the affinity reagent 102 is introduced into the biological sample comprising the target analyte 106. Thus, the affinity reagent 102 may already be specifically bound to the target analyte 106 and the barcode oligonucleotide 110 is amplified in situ.

The barcode oligonucleotide 110, in particular its barcode sequence, is amplified by an isothermal amplification method, preferably by a loop-mediated isothermal amplification method (LAMP). Amplifying the barcode sequence, generates at least one of the amplified barcode sequences 110a. For example, the middle view of Fig. 1 shows ten amplified barcode sequences 110a, to each of which one of the labelling moieties 112 may subsequently hybridise to.

The isothermal amplification method is preferably carried out at a low temperature compared to polymerase chain reactions, which utilise thermocycling with temperatures reaching up to 98° C. This limits any thermal degradation of the biological sample and/or the target analyte 106. For example, the isothermal amplification method is carried out at a constant temperature in a range from 20°C to 75°C. It is preferred, when the isothermal amplification method is carried out at a temperature in a range from 30 °C to 50 °C, in particular at 40 °C. This further reduces a build-up of fluorescent background in the biological sample.

The isothermal amplification method may initiate amplification by providing a set of primers comprising four to six primers. The primers cause the barcode oligonucleotide 110 to loop back on itself. This provides free ends to initiate amplification of the barcode sequence, for example, by a strand displacing polymerase.

After addition of the set of primers, the amplification step is carried out for a period of time in a range from 0.5 min to 60 min. A range from 1 min to 15 min is particularly preferred. This results in an approximately 100- to 10000-fold increase in the number of copies of the barcode sequence. These copies of the barcode sequence may be connected to each other and the barcode sequence to form a continuous sequence.

A particularly preferred isothermal amplification method is provided by Notomi et al, 2000, in particular, Fig. 1 therein and Podushkina et al., 2019, in particular, Fig. 1 therein.

In a preferred embodiment, the barcode oligonucleotide 110 may comprise several different barcode sequences, to each of which a respective complementary sequence 114 with different optically detectable labelling moieties 112 may bind to. The different labelling moieties 112 may have different optical properties. This enables generating a diverse set of marker with different optical properties that may be distinguished in an optical readout.

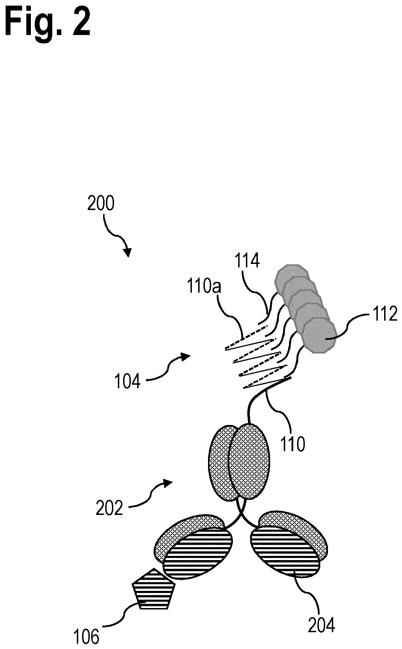

Figure 2 is a schematic view of a marker 200 with an affinity reagent 202. The affinity reagent 202 comprises an amino acid binding backbone 204, for example an antibody. The affinity reagent 202 further comprises the barcode oligonucleotide 110 with the barcode sequence. This enables binding the label 104 to the affinity reagent 102. In particular, complementary oligonucleotides 114 with labelling moieties 112 may be brought into proximity to the affinity reagent 202 and hybridise to (amplified) barcode sequences 110, 110a to generate the label 104. Thus, the barcode oligonucleotide 110, in particular the barcode sequence, of the marker 200 is already amplified to generate amplified barcode sequences 110a.

After the amplifying the barcode sequence and introducing the label 104, an optical readout of the biological sample, in particular the target analyte 106 with the marker 100, 200, may be generated, for example by means of a microscope. The optically detectable labelling moieties 112 of the marker 100, 200 may be detected in the readout and the presence and/or location of the target analyte in the biological sample may be determined.

Identical or similarly acting elements are designated with the same reference signs in all Figures. As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

### Reference signs

- 100, 200: Marker
- 102, 202: Affinity reagent
- 104: Label
- 106: Target analyte
- 108, 204: Binding backbone
- 110: Barcode oligonucleotide
- 110a: Amplified barcode sequence
- 112: Optically detectable labelling moiety
- 114: Complementary oligonucleotide

## Claims

1. An affinity reagent (102, 202) for analysing a biological sample comprising:
a binding backbone (108, 204), and
a barcode oligonucleotide (110) attached to the binding backbone (108, 204),
wherein the binding backbone (108, 204) is configured to specifically bind to a target analyte (106), and
wherein the barcode oligonucleotide (108, 204) comprises at least one barcode sequence and a set of primer sequences configured for isothermal amplification.

2. A method for analysing a biological sample comprising a target analyte (106), the method comprising the following steps:
introducing an affinity reagent (102, 202) comprising at least one barcode sequence, in particular the affinity reagent according to claim 1, into the biological sample,
amplifying within the biological sample the at least one barcode sequence with an isothermal amplification method in order to generate at least one amplified barcode sequence (110a),
introducing a plurality of labels into the biological sample, each label comprising an optically detectable moiety (112) and a complementary oligonucleotide (114) comprising a complementary sequence configured to hybridise to the at least one barcode sequence, and
generating an optical readout of the biological sample.

3. The method according to claim 2, wherein the isothermal amplification method comprises introducing a set of primers into the biological sample.

4. The method according to claim 3, wherein the primers of the set of primers are configured to generate hairpin loops.

5. The method according to claims 3 or 4, wherein the set of primers comprises at least a first primer with a first region of the first primer being configured to hybridise to at least the barcode oligonucleotide (110) towards a 3' end of the barcode oligonucleotide (110), and a second region of the first primer being configured to hybridise to the amplified barcode sequence (110a) downstream of the first region of the first primer.

6. The method according to one of the claims 3 to 5, wherein the set of primers comprises at least a second primer with a first region of the second primer being configured to hybridise to the amplified barcode sequence (110a) towards a 3' end of the amplified barcode sequence (110a), and a second region of the second primer being configured to hybridise to the amplified barcode sequence (110a) downstream of the first region of the second primer.

7. The method according to one of the preceding claims 2 to 6, wherein the amplified barcode sequence (110a) is a continuous sequence comprising multiple copies of the barcode sequence.

8. The method according to one of the preceding claims 2 to 7, wherein the isothermal amplification method comprises introducing a strand displacing polymerase into the biological sample.

9. The method according to one of the preceding claims 2 to 8, wherein the step of amplifying the at least one barcode sequence is carried out at a substantially constant temperature in a range between 20°C to 75°C.

10. The method according to one of the preceding claims 2 to 9, wherein the step of amplifying the at least one barcode sequence is carried out for 0.5 min to 60 min.

11. The method according to one of the preceding claims 2 to 10, wherein the step of introducing an affinity reagent (102, 202) comprises introducing a further affinity reagent comprising at least one further barcode sequence and the step of amplifying within the biological sample comprises amplifying the at least one further barcode sequence with the isothermal amplification method.

12. The method according to one of the preceding claims 2 to 11, wherein the plurality of labels introduced into the biological sample comprises at least a first label with the complementary sequence configured to hybridise to the at least one barcode sequence and at least a second label with a complementary sequence configured to hybridise to the at least one further barcode sequence.

13. The method according to one of the preceding claims 2 to 12, wherein the optically detectable moiety (112) comprises a fluorophore.

14. The method according to one of the preceding claims 2 to 13, wherein in the step of generating the optical readout, at least one of the presence and location of the target analyte (106) is determined in the biological sample.

15. An affinity reagent kit comprising the affinity reagent of claim 1, a set of primers configured to bind to the set of primer sequences, and at least one label comprising a complementary oligonucleotide (114) comprising a complementary sequence configured to hybridise to the barcode sequence.
